**Europäisches Patentamt**

**European Patent Office**

**Office européen des brevets**

(19)

(11) Numéro de publication : **0 117 784
B1**

(12)

# FASCICULE DE BREVET EUROPÉEN

(45) Date de publication du fascicule du brevet :
29.07.87

(21) Numéro de dépôt : **84400182.6**

(22) Date de dépôt : **27.01.84**

(51) Int. Cl.⁴ : **C 10 M159/12**, C 10 M133/58, C 08 F 8/32, C 07 D487/18

(54) Compositions d'additifs dispersants pour huiles lubrifiantes et leur préparation.

(30) Priorité : **04.02.83 FR 8301918**

(43) Date de publication de la demande :
**05.09.84 Bulletin 84/36**

(45) Mention de la délivrance du brevet :
**29.07.87 Bulletin 87/31**

(84) Etats contractants désignés :
**BE DE GB IT NL**

(56) Documents cités :
FR-A- 2 264 084
US-A- 3 962 104
US-A- 4 219 431
**Le dossier contient des informations techniques présentées postérieurement au dépôt de la demande et ne figurant pas dans le présent fascicule.**

(73) Titulaire : **INSTITUT FRANCAIS DU PETROLE
4, Avenue de Bois-Préau
F-92502 Rueil-Malmaison (FR)**

(72) Inventeur : **Denis, Jacques
Les Chênes 18, Chemin Grandes Bruyères
F-69260 Charbonnière Les Bains (FR)**
Inventeur : **Senneron, Michel
Résidence l'Emmendra 23, avenue du Vercors
F-38240 Meylan (FR)**
Inventeur : **Sillion, Bernard
13, rue des Erables
F-78150 Rocquencourt (FR)**

**0 117 784**

## Description

L'invention est relative à de nouvelles compositions dispersantes solubles dans l'huile, un procédé pour leur préparation et les lubrifiants qui contiennent ces compositions. Plus particulièrement, l'invention concerne de nouvelles compositions dispersantes, solubles dans l'huile, produites en faisant réagir un anhydride succinique substitué avec de l'hexaméthylènetétramine et un alkylphénol.

L'un des principaux problèmes rencontrés actuellement dans les lubrifiants de moteurs est dû à la présence inévitable dans le lubrifiant de particules étrangères en suspension, telles que des matières charbonneuses et des sludges provenant des suies, des produits d'altération du carburant et du lubrifiant, et de l'eau.

Or, l'accumulation de ces matières en suspension pose un sérieux problème pour l'efficacité du lubrifiant dans le moteur et il est important d'empêcher l'agglomération et le dépôt de ces matières indésirables sous forme de vernis, de matières charbonneuses dures et de sludges dans les différentes parties du moteur. Depuis plusieurs années, on tente de pallier ces difficultés par l'emploi d'additifs organo-métalliques, tels que par exemple les sulfonates, les phénates ou les salicylates de métaux alcalino-terreux, ou d'additifs organiques, tels que par exemple des polyméthacrylates greffés ou copolymérisés avec des réactifs insaturés azotés, ou des succinimides de polyéthylène-polyamines.

Mais l'utilisation des additifs organo-métalliques est limitée par les risques de dépôts divers d'oxydes métalliques sur les électrodes de bougies dans les moteurs à allumage commandé ; ces dépôts peuvent provoquer du préallumage nuisible pour les moteurs. Les additifs organiques sans cendres connus présentent l'inconvénient d'une efficacité limitée à haute température.

Ainsi, la demande de brevet français FR-A-2 264 084 décrit des additifs détergents pour huiles lubrifiantes qui peuvent résulter de la réaction d'hexaméthylènetétramine et d'un alkylphénol dont au moins une des positions 2, 4 et 6 du noyau aromatique n'est pas substituée.

Le brevet des Etats-Unis US-A-4 219 431 décrit notamment des additifs pour lubrifiants, pouvant consister en les produits de réaction d'une amine choisie parmi les aminoalcanepolyols et les polyalkylène polyamines sur le produit de réaction d'un composé alkénylsuccinique et d'un composé hydroxy-aromatique.

Le brevet des Etats-Unis US-A-3 962 104 décrit des additifs détergents sans cendres pour lubrifiants, consistant en des sels d'ammonium quaternaires, formés par réaction entre une amine tertiaire (par exemple l'hexaméthylènetétramine) et un oxyde d'oléfine en présence d'un excès d'eau, suivie de la réaction avec un composé acide organique, qui peut être entre autres un anhydride succinique substitué (anhydride dodécényl-succinique, ou anhydride polyisobuténylsuccinique) ou un alkyl phénol.

L'invention a pour objet de fournir de nouvelles compositions d'additifs dispersants efficaces dans les lubrifiants et ne présentant pas les inconvénients mentionnés ci-dessus.

D'une manière générale, les compositions d'additifs de l'invention peuvent être définies comme consistant en les produits obtenus par réaction simultanée d'un anhydride succinique substitué, d'hexaméthylènetétramine et d'un alkylphénol.

Plus particulièrement, les anhydrides succiniques substitués utilisables pour préparer les additifs de l'invention sont ceux que l'on obtient :

par condensation d'un anhydride maléique sur un hydrocarbure insaturé, linéaire ou ramifié, oléfinique ou polyoléfinique, possédant au moins une insaturation par molécule, et comprenant de 5 à· 250 atomes de carbone (de préférence, de 60 à 120) ;

par condensation d'un anhydride maléique sur une oléfine ou une polyoléfine halogénée, l'oléfine ou la polyoléfine étant définie comme ci-dessus ; ou

par tout autre procédé connu pour conduire aux produits désignés habituellement par : anhydrides alcénylsucciniques ou anhydrides polyalcénylsucciniques.

Avantageusement, l'anhydride succinique substitué peut résulter de la réaction d'un polymère de monooléfine de 2 à 5 atomes de carbone avec l'anhydride maléique, ledit polymère ayant en général une masse molaire d'environ 500 à 1 600 et plus particulièrement d'environ 700 à 1 300. De tels polymères présentent l'avantage d'une grande disponibilité et d'un faible coût. Comme exemples préférés, on peut citer les polyisobutènes.

Les alkylphénols utilisables pour préparer les compositions d'additifs de l'invention consistent plus particulièrement en des phénols substitués par un ou plusieurs groupements alkyles, linéaires ou ramifiés, ayant de 4 à 12 atomes de carbone, ces groupements alkyles étant situés en ortho et/ou en para de la fonction phénol. Comme exemples préférés d'alkylphénols, on peut citer : le p-nonylphénol, l'o-nonylphénol, le p-dodécylphénol, l'o-dodécylphénol, les ditertiobutyl-2,4 et ditertiobutyl-2,6 phénols, le p-hexylphénol, le p-heptylphénol et le p-octylphénol.

La réaction mise en œuvre pour produire les additifs de l'invention est réalisée par mélange des réactifs définis plus haut en des proportions correspondant généralement à un rapport molaire de l'anhydride succinique substitué à l'alkylphénol d'environ 1/1 à 2/1, et à un excès d'hexaméthylènetétramine par rapport à l'anhydride succinique substitué. La quantité d'hexaméthylènetétramine mise en jeu correspond en général à un nombre de 1,2 à 2 fonctions amines par mole d'anhydride succinique substitué, c'est-à-dire de 0,3 à 0,5 mole d'hexaméthylènetétramine par mole d'anhydride succinique

2

substitué.

La réaction s'effectue en général à une température de 120 à 250 °C et, de préférence, de 180 à 210 °C, pendant une durée de 1 à 3 heures et, de préférence, de 1 h 30 à 2 h 30 minutes. Elle peut être conduite soit en l'absence de solvant, soit dans une quantité d'huile telle que le produit final contienne environ 50 % en poids de matière active.

Lorsque l'on opère en l'absence de solvant, le produit de la réaction pourra être ultérieurement solubilisé dans de l'huile, de façon à obtenir une solution de viscosité convenable ; on peut procéder soit à une filtration, soit à un lavage à l'eau, soit encore à un dégazage par barbotage de gaz inerte de façon à éliminer l'hexaméthylènetétramine non réagie et ses produits de décomposition.

Généralement, les compositions d'additifs de l'invention peuvent être utilisées dans les lubrifiants seules ou en combinaison avec d'autres additifs conventionnels. Comme additifs dispersants dans les huiles, elles peuvent être utilisées dans des proportions comprises entre 0,1 et 20 % en poids du lubrifiant, suivant l'usage auquel le lubrifiant est destiné et suivant la présence ou l'absence d'autres additifs spécialement dispersants et/ou détergents. Ordinairement, leur proportion sera comprise entre 1 et 10 % en poids du lubrifiant.

Les compositions de l'invention peuvent en effet être incorporées à diverses huiles de base naturelles, synthétiques ou mixtes, utilisées à des fins diverses, telles que les lubrifiants pour moteurs à combustion interne à allumage commandé ou à allumage par compression, (comme par exemple les moteurs d'automobiles ou de camions, les moteurs deux temps, les moteurs d'avion à piston, les moteurs marins ou encore les Diesels ferroviaires). De plus, les fluides de transmission automatique, d'engrenage, de travail des métaux, d'hydraulique et les graisses peuvent aussi bénéficier de l'incorporation des additifs de l'invention.

Normalement, les compositions de l'invention sont utilisées en mélange avec d'autres additifs conventionnels. Ceux-ci comprennent les produits phosphorés ou soufrés agents extrême-pression, les détergents organométalliques tels que les phénates sulfures, les sulfonates et les salicylates de métaux alcalino-terreux, les dispersants sans cendres, les polymères épaississants ainsi que les anticongelants, les inhibiteurs d'oxydation, les agents anticorrosifs, antirouille et antimousse et divers.

Les exemples suivants illustrent l'invention, sans en limiter la portée.

## Exemple 1

Dans un ballon muni d'un agitateur, d'un thermomètre et d'un réfrigérant ascendant, on introduit 20 g d'un anhydride polyisobuténylsuccinique contenant 0,0715 fonction anhydride par 100 g (soit l'équivalent de 0,0143 fonction anhydride), puis 3,14 g de p-nonylphénol (0,0143 mole), et 0,67 g (0,0048 mole) d'hexaméthylènetétramine. On porte l'ensemble agité 1 heure 30 minutes à 190 °C. Le résultat de la réaction se présente sous forme d'un liquide limpide et visqueux jaune foncé ; c'est l'additif I.

Cet additif est solubilisé à raison de 5 % en poids dans une huile minérale 200 Neutral Solvant, et son efficacité dispersante est évaluée par l'essai à la tache sur papier filtre, en présence de matière charbonneuse issue d'une huile usagée de moteur Diesel. Le rapport entre les diamètres de la tache noire et de l'auréole d'huile est de 0,67.

## Exemple 2

En suivant les mêmes conditions opératoires que celles de l'exemple 1, on condense 20 g de l'anhydride polyisobuténylsuccinique utilisé dans l'exemple 1 (soit 0,0143 équivalent gramme d'anhydride), 3,74 g (0,0143 mole) de p-dodécylphénol et 0,67 g (0,0048 mole) d'hexaméthylènetétramine. On obtient ainsi l'additif II ; le rapport des diamètres lors d'un essai à la tache conduit de façon similaire à celle décrite dans l'exemple 1 est de 0,65.

## Exemple 3

On procède de la même façon que dans l'exemple 1, mais on remplace le p-nonylphénol par 2,95 g (0,0143 mole) de ditertiobutyl-2,6 phénol, et l'on obtient l'additif III. Après dilution à 5 % en poids dans une huile minérale 100 Neutral, la solution obtenue est filtrée. L'essai à la tache donne un rapport de 0,60.

## Exemple 4

On chauffe pendant 1 heure 30 à 190 °C un mélange constitué par : 40 g de l'anhydride polyisobuténylsuccinique utilisé dans l'exemple 1 (soit l'équivalent de 0,0286 fonction anhydride), 3,14 g de p-nonylphénol (0,0143 mole), 1,4 g (0,01 mole) d'hexaméthylènetétramine et 45 g d'huile minérale 100 Neutral Solvant. Après filtration sous pression, on obtient une solution d'additif IV limpide et de couleur brun clair. Sa valeur de dispersivité obtenue par essai à la tache est de 0,67.

## Exemple 5

**0 117 784**

Les produits préparés dans les exemples 1 à 4 ont été soumis à un essai de cokéfaction qui permet d'évaluer la stabilité thermique de l'additif et son efficacité dispersante. Cet essai consiste à projeter l'huile additivée pendant 20 heures sur la paroi externe d'un Becher métallique chauffé à 310 °C, selon une méthode ANTAR. A l'issue de cet essai, on détermine le poids de dépôt collé à la surface métallique et on examine l'aspect de ce dépôt sur le fond et sur la jupe du Becher. Les résultats, comparés à ceux obtenus avec un additif industriel de type bis(alcénylsuccinimide), sont donnés dans le tableau A suivant. L'huile est constituée par un mélange de 350 Neutral et de Bright Stock dans un rapport pondéral 92/8, et contient dans chaque essai 5 % en poids de l'additif à tester et 1 % en poids d'inhibiteur d'oxydation de type dialkyldithiophosphate de zinc.

Tableau A

Essais de cokéfaction

| Additif dispersant | Réf. | additif I | additif II | additif III | additif IV |
|---|---|---|---|---|---|
| Poids de dépôt en mg | 660 | 630 | 600 | 560 | 580 |
| Aspect du fond/10 | 4,5 | 4,5 | 5 | 5 | 5 |
| Aspect de la jupe/10 | 6,5 | 8 | 8 | 7,5 | 7,5 |
| Consommation d'huile en g | 70 | 75 | 72 | 80 | 76 |

Exemple 6

Dans cet exemple, les additifs dispersants I et IV selon l'invention sont essayés sur moteur Diesel Petter $AV_1$, afin d'évaluer non seulement leur efficacité dispersante mais aussi leur stabilité thermique, et les comparer à celles de l'additif de référence décrit dans l'exemple 5. La méthode utilisée avec le moteur Petter $AV_1$ est normalisée sous le n° IP 175/69 ; elle dure 120 heures.

Les résultats d'essais sont donnés dans le tableau B suivant :

Tableau B

Essais sur moteur Petter $AV_1$

| Additif dispersant | référence | additif I | additif IV |
|---|---|---|---|
| Cotation des dépôts sur le piston | | | |
| moyenne cordons/gorges/10 | 8,85 | 9,2 | 9,0 |
| fond | 6,5 | 7,5 | 7,2 |
| Cotation du sludge sur les pièces froides du moteur | | | |
| couvercle des poussoirs | 6,4 | 8,5 | 8,1 |
| cache culbuteurs | 8,2 | 9,0 | 9,6 |

Ces résultats traduisent un effet dispersant et une stabilité thermique améliorés pour les compositions d'additifs de l'invention.

**Revendications**

1. Composition d'additif à effet dispersant dans les huiles lubrifiantes, caractérisée en ce qu'elle consiste en le produit de la réaction simultanée d'au moins un anhydride succinique substitué, d'au moins un alkylphénol et d'hexaméthylènetétramine, ladite réaction étant réalisée avec une quantité molaire de l'anhydride succinique substitué de 1/1 à 2/1 par rapport à la quantité molaire d'alkylphénol et une quantité d'hexaméthylènetétramine, exprimée en fonctions amines, de 1,2 à 2 par rapport à la

4

0 117 784

quantité molaire d'anhydride succinique substitué.

2. Composition d'additif selon la revendication 1, caractérisée en ce que ledit anhydride succinique substitué est choisi parmi les produits obtenus par condensation d'un anhydride maléique sur un hydrocarbure insaturé linéaire ou ramifié, oléfine ou polyoléfine, présentant au moins une insaturation par molécule et comprenant de 5 à 250 atomes de carbone ; les produits obtenus par condensation d'un anhydride maléique sur une oléfine ou une polyoléfine halogénée, l'oléfine ou la polyoléfine étant définie comme ci-dessus ; et les produits obtenus par tous autres procédés connus pour conduire à des anhydrides alcénylsucciniques ou à des anhydrides polyalcénylsucciniques.

3. Composition d'additif selon la revendication 1 ou 2, caractérisée en ce que ledit anhydride succinique substitué résulte de la réaction d'un polymère de monooléfine en $C_2$-$C_5$ avec l'anhydride maléique, ledit polymère présentant une masse molaire de 500 à 1 600.

4. Composition d'additif selon la revendication 3, caractérisé en ce que ledit polymère est un polyisobutène.

5. Composition d'additif selon l'une des revendications 1 à 4, caractérisée en ce que ledit alkylphénol consiste en un phénol substitué par au moins un groupement alkyle, linéaire ou ramifié, de 4 à 12 atomes de carbone, situé en ortho et/ou en para de l'hydroxyle.

6. Composition d'additif selon la revendication 5, caractérisée, en ce que ledit alkylphénol est choisi parmi les o- et p-nonylphénols, les o- et p-dodécylphénols, les ditertiobutyl-2,4 et 2,6 phénols, le p-hexylphénol, le p-heptylphénol et le p-octylphénol.

7. Composition d'additif selon l'une des revendications 1 à 6, caractérisée en ce que ladite réaction est effectuée à une température de 120 à 250 °C pendant un temps de 1 à 3 heures.

8. Composition d'additif selon l'une des revendications 1 à 7, caractérisée en ce que ladite réaction est effectuée au sein d'une huile.

9. Composition lubrifiante caractérisée en ce qu'elle renferme une proportion majeure d'une base lubrifiante et, à titre d'additif, au moins une composition selon l'une des revendications 1 à 8, en une proportion de 0,1 à 20 % en poids par rapport à ladite base lubrifiante.


### Claims

1. An additive composition having a dispersing effect in lubricating oils, characterized in that it consists of the product obtained by simultaneously reacting at least one substituted succinic anhydride with at least one alkylphenol and with hexamethylenetetramine, the reaction being performed with a molar amount of the substituted succinic anhydride of 1/1 to 2/1 with respect to the molar amount of the alkylphenol and with an amount of hexametylenetetramine, expressed as amine functions, of 1.2 to 2 with respect to the molar amount of the substituted succinic anhydride.

2. An additive composition according to claim 1, characterized in that the substituted succinic anhydride is selected from the products obtained by condensing a maleic anhydride with an unsaturated, linear or branched, olefinic or polyolefinic, unsaturated hydrocarbon having at least one unsaturation per molecule and comprising from 5 to 250 carbon atoms ; the products obtained by condensing a maleic anhydride with a halogenated olefin or polyolefin of the above-defined type ; and the products obtained by any other process known as producing alkenylsuccinic anhydrides or polyalkanylsuccinic anhydrides.

3. An additive composition according to claim 1 or 2, characterized in that said substituted succinic anhydride results from reacting a polymer of $C_2$-$C_5$ monoolefin with maleic anhydride, said polymer having a molecular weight from 500 to 1 600.

4. An additive composition according to claim 3, characterized in that said polymer is polyisobutene.

5. An additive composition according to one of claims 1 to 4, characterized in that said alkylphenol is a phenol substituted with at least one linear or branched alkyl group, having from 4 to 12 carbon atoms, in ortho and/or in para position with respect to the hydroxyl group.

6. An additive composition according to claim 5, characterized in that said alkylphenol is selected from o- and p-nonylphenols, o- and p-dodecylphenols, 2,4- and 2,6-ditert-butyl phenols, p-hexylphenol, p-heptylphenol and p-octylphenol.

7. An additive composition according to one of claims 1 to 6, characterized in that said reaction is performed at a temperature from 120 to 250 °C for 1 to 3 hours.

8. An additive composition according to one of claims 1 to 7, characterized in that said reaction takes place in oil.

9. A lubricant composition characterized in that it contains a major proportion of a lubricant base and, as an additive, at least one composition according to one of claims 1 to 8, in a proportion from 0.1 to 20 % by weight with respect to said lubricant base.


### Patentansprüche

1. Dispergierzusatz-Zusammensetzung für Schmieröle, dadurch gekennzeichnet, daß sie aus dem Produkt der gleichzeitigen Umsetzung von zumindest einem substituierten Bernsteinsäureanhydrid, zumindest einem Alkylphenol und Hexamethylentetramin besteht, wobei dieses Umsetzung mit einer molaren Menge an substituiertem Bernsteinsäureanhydrid von 1 : 1 bis 2 : 1, bezogen auf die molare Menge des Alkylphenols und einer Menge Hexamethylentetramin, ausgedrückt als Aminfunktionen, von 1,2 bis 2, bezogen auf die molare Menge des substituierten Bernsteinsäureanhydrids durchgeführt wird.

2. Zusatzzusammensetzung nach Anspruch 1, dadurch gekennzeichnet, daß das substituierte Bernsteinsäureanhydrid aus den Produkten gewählt wird, die erhalten sind durch Kodensation eines Maleinsäureanhydrids mit einem linearen oder verzweigten ungesättigten Kohlenwasserstoff, Olefin oder Polyolefin, die zumindest eine Unsättigung pro Molekül aufweisen und 5 bis 250 Kohlenstoffatome enthalten, aus den Produkten, die durch Kondensation eines Maleinsäureanhydrids mit einem halogenierten Olefin oder Polyolefin erhalten sind, wobei das Olefin oder Polyolefin wie oben definiert sind, und aus den Produkten, die erhalten sind durch alle anderen bekannten Verfahren zur Herstellung von Alkenylbernsteinsäureanhydriden oder Polyalkenylbernsteinsäureanhydriden.

3. Zusatzzusammensetzung nach Anspruch 1 oder 2, dadurch gekennzeichnet, daß sich das substituierte Bernsteinsäureanhydrid aus der Umsetzung eines Polymeren eines $C_2$-$C_5$ Mono-olefins mit Maleinsäureanhydrid ergibt, wobei dieses Polymere eine molare Masse von 500 bis 1 600 aufweist.

4. Zusatzzusammensetzung nach Anspruch 3, dadurch gekennzeichnet, daß das Polymere ein Polyisobuten ist.

5. Zusatzzusammensetzung nach einem der Ansprüche 1 bis 4, dadurch gekennzeichnet, daß das Alkylphenol aus einem Phenol besteht, das durch zumindest eine lineare oder verzweigte $C_4$-$C_{12}$ Alkylgruppe substituiert ist, die in Ortho- und/oder Parastellung zur Hydroxylgruppe sitzt.

6. Zusatzzusammensetzung nach Anspruch 5, dadurch gekennzeichnet, daß das Alkylphenol gewählt ist aus den Verbindungen o- und p-Nonylphenole, o- und p-Dodecylphenole, 2,4- und 2,6-Ditertbutylphenole, p-Hexylphenol, p-Heptylphenol und p-Octylphenol.

7. Zusatzzusammensetzung nach einem der Ansprüche 1 bis 6, dadurch gekennzeichnet, daß die Umsetzung bei einer Temperatur von 120 bis 250 °C während einer Zeitspanne von 1 bis 3 Stunden bewirkt wird.

8. Zusatzzusammensetzung nach einem der Ansprüche 1 bis 7, dadurch gekennzeichnet, daß die Umsetzung in einem Öl bewirkt wird.

9. Schmiermittel-Zusammensetzung, dadurch gekennzeichnet, daß sie einen größeren Teil einer Schmiermittelbasis und als Zusatz zumindest eine Verbindung nach einem der Ansprüche 1 bis 8 in einer Menge von 0,1 bis 20 Gew.-%, bezogen auf die Schmiermittelbasis, enthält.